Europäisches Patentamt

European Patent Office

Office européen des brevets

(19)

(11) Numéro de publication: **0 232 678 B1**

# FASCICULE DE BREVET EUROPEEN

(12)

(45) Date de publication de fascicule du brevet:
03.04.91

(51) Int. Cl.⁵: **A61B 17/22**

(21) Numéro de dépôt: 86810616.2

(22) Date de dépôt: 31.12.86

(54) **Dispositif chirurgical.**

(30) Priorité: 31.12.85 CH 55/86
30.08.86 CH 3466/86

(43) Date de publication de la demande:
19.08.87 Bulletin 87/34

(45) Mention de la délivrance du brevet:
03.04.91 Bulletin 91/14

(84) Etats contractants désignés:
AT BE CH DE ES FR GB GR IT LI LU NL SE

(56) Documents cités:
EP-A- 0 147 192       CH-A- 587 044
DE-A- 3 337 258       DE-A- 3 421 390
US-A- 2 688 968       US-A- 2 982 582
US-A- 4 331 422

(73) Titulaire: **Neracher, Arnold**
**31, chemin du Nant d'Aisy**
**CH-1247 Anières(CH)**

(72) Inventeur: **Neracher, Arnold**
**31, chemin du Nant d'Aisy**
**CH-1247 Anières(CH)**

(74) Mandataire: **Charbonnier, Georges R.**
**8, Avenue Peschier**
**CH-1206 Genève(CH)**

EP 0 232 678 B1

## Description

La présente invention a pour objet un dispositif chirurgical utilisable notamment en angioplastie.

Ce dispositif est constitué par un cathéter comprenant un tuyau souple et un conduit métallique flexible s'étendant dans ce tuyau, par une source de liquide sous pression relié à l'une des deux extrémités de ce conduit, et par une buse formant la seconde extrémité de ce conduit et dont le diamétre est compris entre 20 et 70 microns, le tout de manière que la buse projette un jet de liquide animé d'une vitesse relativement élevée.

On connait un dispositif de ce type, décrit dans le brevet DE - A - 3 421 390 , destiné à éliminer un dépôt organique obstruant partiellement ou totalement un vaisseau du corps humain, dans lequel la vitesse initiale du jet est inférieure à 300 m/s.

Avec ce dispositif la vitesse du jet diminue très rapidement au contact du liquide organique par suite de l'entrainement par frottement d'une quantité importante de ce liquide. Dans ces circonstances la massec en mouvement augmente, son front s' élargit et son impact sur la matière du dépôt se traduit par une poussée qui tend à arracher le dépôt.

Le dispositif selon la présente invention permet d' obtenir, au contraire, dans ce cas, une élimination parfaitement contrôlée de ce dépôt en procédant par coupe fine, incision ou perçage.

Le chirurgien peut ainsi attaquer progressivement le dépôt, le diviser avec une très grande précision, enlever successivement des parties de celui-ci, l' éliminer partiellement ou totalement, et cela sans toucher à la paroi du vaisseau et par conséquent sans créer aucune lésion à ce niveau. le dispositif selon la présente invention est caractérisé par le fait qu'il comprend une source de liquide alimentant la buse à une pression égale ou supérieur à $6.10^7$ Pa et une buse formant un jet dont la vitesse initiale est égale ou supérieure au nombre de Mach.

En mettant en oeuvre ce dispositif pour éliminer un dépôt organique obstruant partiellement ou totalement un vaisseau du corps humain,on constate à la sortie de la buse en régime supersonique la formation d'un cône de cavitation de quelques microns d'épaisseur et de 2-3 mm de longueur,résultant du gradient de tension de cisaillement entre le jet animé d'une vitesse supersonique et le liquide organique (au repos) entourant celui-ci.

La cavitation prend naissance lorsque la diminution de pression dans le liquide est inférieure à la tension de vapeur de celui-ci.

Cette enveloppe de vapeur ,qui isole dynamiquement le jet supersonique du liquide organique environnant,se présente sous la forme d'un cône,de quelques microns d'épaisseur,dans lequel la cavitation est particulièrement intense.

En modifiant et réglant la position de la buse,le chirurgien Peut amener le sommet de ce cône au contact du dépôt ce qui provoque,par érosion,un enlèvement pratiquement ponctuel de matière.

En faisant décrire à ce point d'impact une trajectoire déterminée appropriée il peut enlever tout ou partie du dépôt obstruant le vaisseau sans toucher à la paroi de celui-ci.

Le dessin ci-annexé représente, schématiquement et à titre d'exemple, plusieurs formes d'exécution de l'objet de l'invention destineés á l'angioplastie.

La figure 1 est une vue schématique d'une première forme d'exécution.

La figure 2 est une vue en coupe longitudinale de l'extrémité du dispositif figurant dans le bloc en trait mixte de la figure 1.

La figure 3 est une vue en coupe de la deuxième forme d'exécution.

La figure 4 est une vue en coupe, à plus grande échelle, de la buse de cette deuxième forme d'exécution.

Les figures 5 à 10 sont des vues analogues à la figure 4, de buses de six autres fcrmes d'exécution présentant, dans leur ensemble, la même structure que celle du dispositif illustré à la figure 3.

La figure 11 est une vue en coupe d'une autre forme d'exécution comprenant une buse dite à mémoire de forme.

La figure 12 est une vue en coupe d'une autre forme d'exécution dont la buse comporte un ballonet de dilatation intégré.

La figure 13 est une vue analogue à la figure 12 illustrant le fonctionnement de cette dernière forme d'exécution.

Les figures 14 et 15 sont des vues analogues à la figure 4 de buses de deux autres formes d'exécution présentant dans leur ensemble la même structure que celle du dispositif illustré à la figure 3.

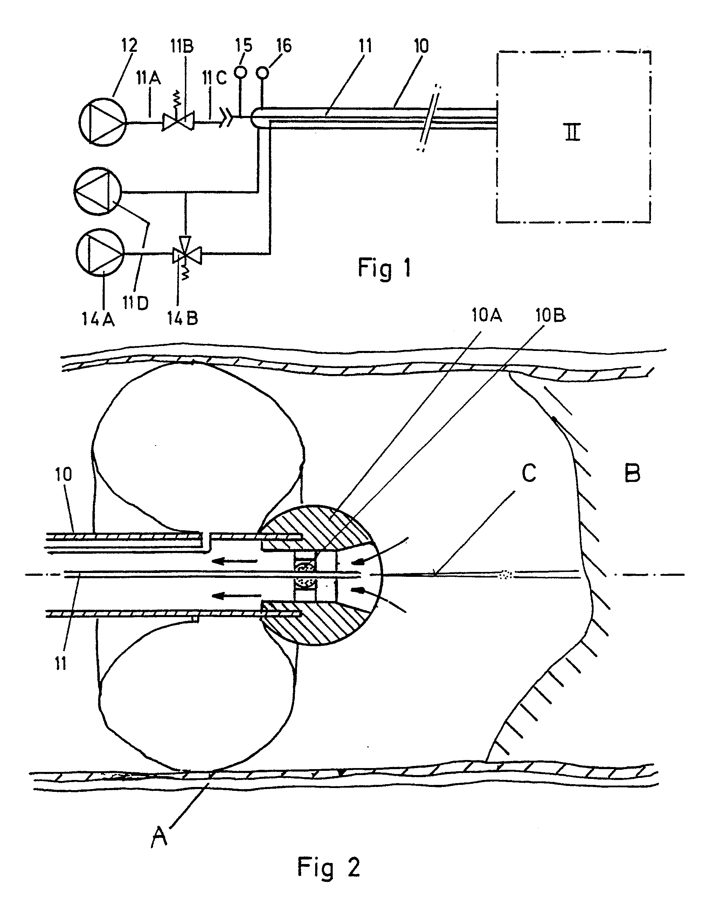

La forme d'exécution représentées aux figures 1 et 2 comprend un cathéter constitué par un tuyau souple 10, en matière synthétique, un conduit métallique souple 11, par exemple en métal ductil ou en matériau isolant précontraint comme le polycarbonate/fibres Kevlar (R), capable d'une bonne souplesse tout

2

EP 0 232 678 B1

en pouvant résister à des pressions de l'ordre de 2.10$^8$ Pa, s'étendant à l'intérieur du tuyau 10, une pompe haute pression 12, une buse 13 fixée à l'extrémité du conduit 11 par soudure ou collage, un ballonet gonflable 14, deux poignées 15 et 16 permettant de déplacer axialement et angulairement, de façon indépendante, le tuyau 10 et le conduit 11.

La pompe 12 alimente le conduit 11 en liquide à haute-pression par l'intermédiaire d'une conduite 11$_A$, d'un régulateur de pression 11$_B$, et d'un raccord rapide 11$_C$.

Lorsque le régulateur 11$_B$ est en service, la buse 13 est alimentée en liquide haute-pression et projette, approximativement dans l'axe du vaisseau A en direction du dépôt B, un jet supersonique C.

Le ballonet 14 assure le centrage de la buse 13 et empêche le liquide du jet de remonter dans le vaisseau A.

Ce ballonet 14 est gonflé au moyen d'une pompe 14$_A$ munie d'un régulateur de pression 14$_B$ par l'intermédiaire d'un tuyau 14$_C$.

Pour faire avancer la buse 13 durant l'élimination du dépôt B, on agit sur les poignées 15 et 16.

L'extrémité du tuyau 10 est munie d'un corps sphérique 10$_A$ présentant un passage axial et d'une rotule 10$_B$ traversée par le conduit 11 et permettant d'orienter le jet C jusqu'à 10° par rapport à l'axe du vaisseau A.

Le fonctionnement du dispositif décrit est le suivant :

Après avoir introduit le cathéter dans le vaisseau A et l'avoir fait avancer dans celui-ci, conjointement avec le conduit 11, au moyen des poignées 15 et 16 jusqu'à ce que la buse 13 se trouve à proximité du dépôt B, selon une orientation appropriée, on met en service la pompe 12 et le régulateur 11$_B$ de manière que le jet C atteigne une vitesse supersonique.

Ce jet vient frapper le dépôt B et, selon sa vitesse et le type de buse utilisée, le détruit, le désagrège ou le démantèle par coupe, incision, ablation, arrachage ou perçage.

Le liquide qui remplit la zone limitée par le ballonet 14 est évacué avec la matière détachée du dépôt A à travers le corps 10$_A$ et le tuyau 11 sous l'action d'une pompe 11$_D$.

Pour obtenir un jet supersonique présentant les caractéristiques P = 10$^8$ Pa ; V$_i$ = 300m/s ; débit = 10$^{-6}$ m$^3$/s ; E$_{cin}$ = 45 j/s on utilisera avantageusement :

- un conduit 10 en acier 316 L

$$D_{int.} = 2.10^{-4} \text{ m} \qquad D_{ext.} = 3.10^{-4} \text{ m}$$

- une buse 13

$$D_{int.} = 6.10^{-5} \text{ m} \qquad D_{ext.} = 3.10^{-4} \text{ m}$$

$D_{int.}$ = diamètre intérieur
$D_{ext.}$ = diamètre extérieur

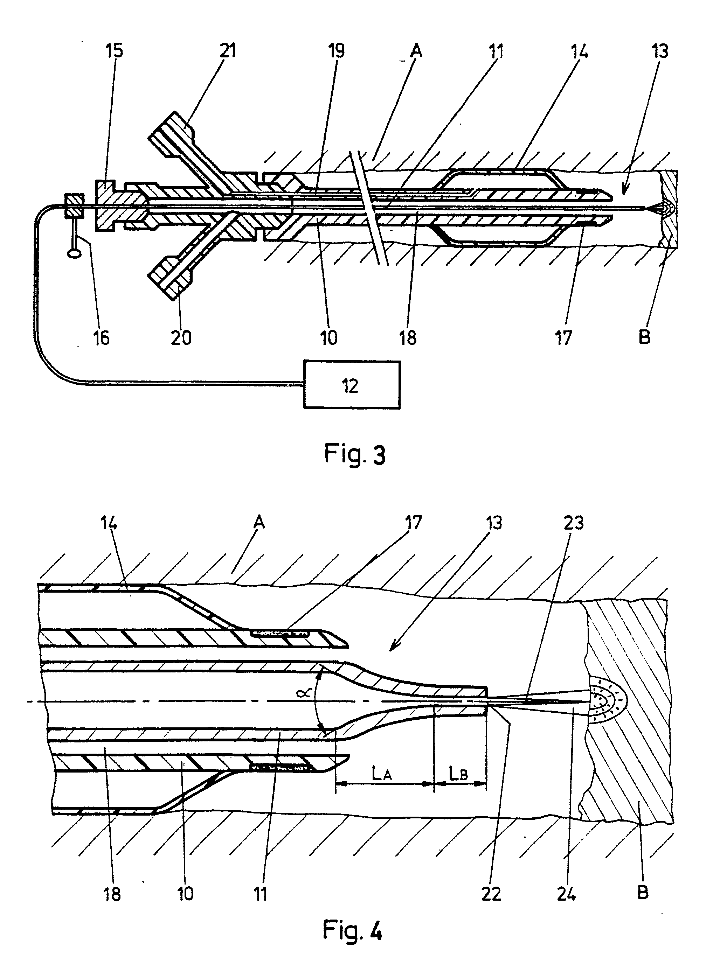

Les formes d'exécution représentées aux figures 3 à 15 ont été conçues et réalisées de manière à résoudre le problème technologique posé, au niveau de la buse 13, par la pression très élevée, supérieure à 6.10$^7$ Pa qu'elle doit supporter et par le diamètre, extrêmement faible, compris entre 20 et 70 $\mu$ de son orifice.

Pour résoudre ce problème, le buses 13 de ces différentes formes d'exécution ont été réalisées, au moins en partie, par l'extrémité du conduit 11.

Le dispositif représenté aux figures 3 et 4 comprend, comme la première forme d'exécution, un tuyau souple 10, un conduit métallique souple 11, une source de liquide haute-pression 12, une buse 13, un ballonet 14, deux poignées 15 et 16, un collier radio-opaque 17.

Le passage 18 formé par l'intérieur du tuyau 10 et l'extérieur du conduit 11, et un canal 19 ménagé dans l'intérieur du tuyau 10 et débouchant dans le ballonet 14 sont destinés à être reliés respectivement en 20 et 21 21 à une pompe à vide et à une source de fluide sous pression.

Le conduit 11, réalisé en un métal ou en un alliage inoxydable, présente un diamètre extérieur de 0,3 mm et un diamètre intérieur de 0,2 mm. Sa résistance à la rupture est de l'ordre de 2200 N/mm$^2$.

La buse 13 est constituée exclusivement par l'extrémité du conduit 11 formée de deux tronçons : un premier tronçon de longueur L$_A$ dont la section intérieure décroit, de façon continue, et un second tronçon, de longueur L$_B$ dont la section intérieure est constante.

La longueur L$_A$ est égale à environ 800 $\mu$, la longueur L$_B$ à environ 160 $\mu$, et le diamètre de l'orifice 22 de 40$\mu$.

L'angle $\alpha$ est de 15° et le rapport entre les sections du conduit 11 et de l'orifice 22 est d'environ 100.

Le coefficient d'orifice Ψ, apparaissant dans la relation

3

EP 0 232 678 B1

$$V = \psi \sqrt{2gH}$$

où H mesure la pression du liquide en amont de la buse et V la vitesse du jet dans le plan de l'orifice 22, est égal, pour cette buse, à 0,98. Pour une pression de $6.10^7$ Pa, la vitesse du jet est de 350 m/s. Cette vitesse atteint 600 m/s pour une pression de $2.10^8$ Pa.

Le jet supersonique est formé d'un dard 23, très effilé et incisif, et d'une zone de turbulence 24. La longueur du dard 23 est environ égale à 5 à 50 fois le diamètre de l'orifice 22.

Ce dispositif s'utilise comme suit :

On introduit le tuyau 10 dans le vaisseau A obstrué par un dépôt B jusquà ce que le collier 17 se trouve à proximité immédiate de ce dépôt à éliminer, puis on gonfle le ballonnet 14 au moyen du fluide entrant en 21; on introduit ensuite le conduit 11 dans le tuyau 10 jusqu'à ce que la buse 13 atteigne le niveau du collier 17.

On alimente le conduit 11 en liquide sous pression de manière que le dard 23 du jet supersonique attaque le dépôt B selon un impact bien déterminé et commence à provoquer sa destruction.

En agissant sur la poignée 16, on déplace la buse 13 axialement et angulairement afin d'agir avec le maximum d'efficacité sur le dépôt. Pour pouvoir maitriser le mieux possible les changements de positions et l'orientation du jet et notamment du dard 23, ainsi que pour suivre la progression de l'élimination du dépôt, on utilisera avantageusement un liquide contenant un produit de contraste aux rayons X.

Durant l'action **du jet,** le liquide chargé de particules arrachées au dépôt, est aspiré par la pompe à vide branchée en 20 et évacué vers l'extérieur à travers le passage 18.

On notera que l'action du dard 23 peut s'opérer, en fonction de ses caractéristiques, par ablation, incision, érosion, coupe, etc.

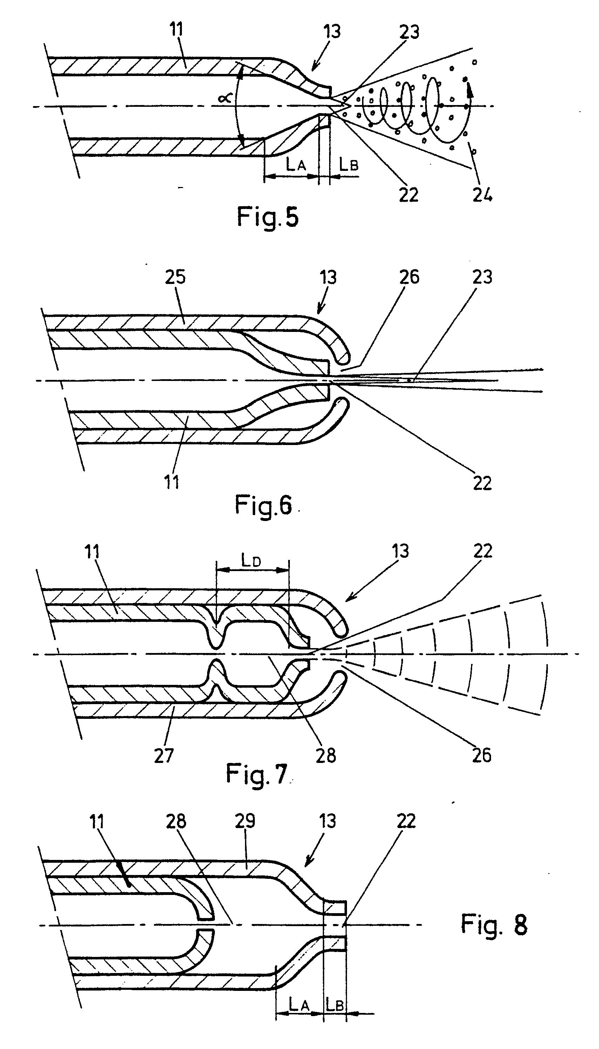

La buse 13 représentée à la figure 5 est constituée exclusivement, comme celle de la figure 4,par l'extrémité, formée de deux tronçons, du conduit métallique 11. Le premier tronçon, de longueur $L_A$ , est tronconique, et le second tronçon, de longueur $L_B$, est cylindrique.

Le diamètre intérieur du second tronçon est de 70 $\mu$ et l'angle $\alpha$ est égal à 60$^\circ$.

La longueur $L_A$ est approximativement égale au diamètre intérieur du conduit 11 et la longueur $L_B$ est plus petite ou égale à la moitié du diamètre de l'orifice 22.

Le coefficient d'orifice $\psi$ de cette buse est égal à 0,8.

Pour une pression d'alimentation de l'ordre de $10^8$ Pa, le dard 23 s'étend sur une longueur de l'ordre de 2 à 10 fois le diamètre de l'orifice 22. Il est enveloppé par une zone turbulente 24 engendrée par la rugosité de la paroi intérieure du tronçon cylindrique. Cette turbulence est à l'origine d'un tourbillon favorisant une action de cavitation. Le conduit 11 est en acier inoxydable est son extrémité est trempée.

La buse 13 représentée à la figure 6 est destinée à être alimentée par un liquide à une pression relativement élevée de l'ordre de 3 à 15. $10^8$ Pa. Elle est constituée par deux éléments : L'extrémité du conduit métallique souple 11 et l'extrémité d'un tube de force 25 s'étendant sur toute la longueur du conduit 11.

Ces deux éléments sont réalisés en alliages métalliques présentant respectivement des résistances à l'éclatement de l'ordre de 2000 N/mm$^2$ et 2700 N/mm$^2$.

Le tube 25 est arrondi au voisinage de son ouverture 26 pour éviter tout risque d'accrochage avec la paroi du vaisseau et le diamètre de cette ouverture est environ le double de celui de l'orifice 22. Avec une pression du liquide de l'ordre de 15. $10^8$ Pa, la vitesse supersonique du jet peut atteindre 1700 m/s ce qui représente, pour un diamètre de 40 $\mu$ de l'orifice 22 un débit de 2 cm$^3$/s et une puissance d'environ 2 kW.

Pour tenir compte de l'énergie calorifique non négligeable dissipée par cette buse, on travaillera en régime discontinu.

La buse 13 représentée à la figure 7 est également constituée par deux éléments : l'extrémité du conduit métallique 11 et l'extrémité d'une gaine de protection 27, en métal ou en matière plastique.

Cette buse 13 présente une chambre de résonance ou cavité résonnante 28, formée par un rétrécissement du conduit 11.

Le liquide arrive en amont de cette chambre 28 à une pression constante relativement basse, de l'ordre de 6. $10^7$ Pa , et sort par l'orifice 22 à une vitesse variant de façon périodique.

La fréquence de cette variation dépend de la longueur $L_D$ de la chambre 28 et de la vitesse de propagation de l'onde élastique dans le liquide. Le jet supersonique à vitesse variable de cette buse est particulièrement favorable pour l'élimination des dépôts organiques durs.

La buse 13 représentée à la figure 8 est une variante de celle représentée à la figure 7.

Elle est constituée par l'extrémité du conduit métallique souple 11 et par l'extrémité d'un tube de force

4

métallique 29.

Ces extrémités sont décalées de manière à ménager entre elles une chambre de résonance 28.

L'orifice 22 de cette buse 13 est constitué par l'ouverture du tube de force 29.

L'extrémité de ce tube 29 présente un tronçon tronconique de longueur $L_A$ et un tronçon cylindrique de longueur $L_B$ se situant approximativement dans le même rapport que les longueurs correspondantes de la buse 13 représentée à la figure 3.

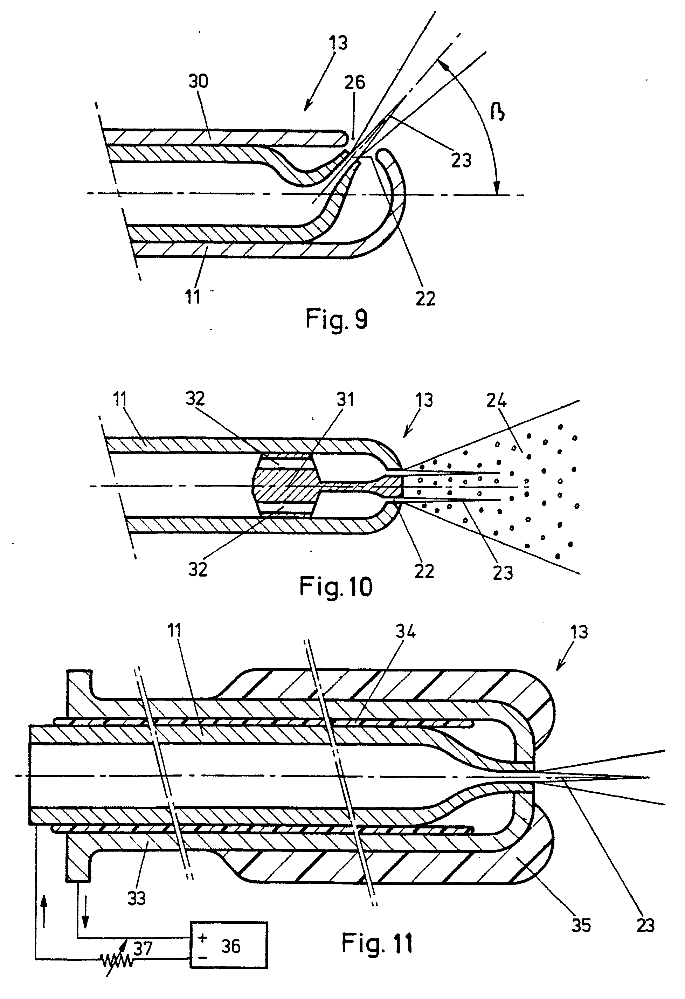

La buse représentée à la figure 9 est une variante de celle illustrée à la figure 6.

Elle est constituée par l'extrémité du conduit métallique 11 et par l'extrémité d'un tube de protection 30 en métal ou en matière plastique.

L'extrémité du conduit 11 est coudée de manière que l'axe du tronçon cylindrique et de l'orifice 22 forme un angle $\beta$ de 60° avec l'axe du vaisseau A. En variante, cet angle $\beta$ pourrait être différent de 60° et être compris entre 0 et 120°.

L'ouverture 26 à bord arrondi du tube 30 est situé en regard de l'orifice 22.

La buse 13 représentée à la figure 10 est constituée par l'extrémité du conduit métallique 11 et par une pièce métallique 31 disposée à l'intérieur du conduit 11.

Cette pièce 31 comporte des passages 32 pour le liquide et une extension qui se termine à l'intérieur de l'orifice 22.

Le jet supersonique produit par cette buse se compose d'un dard annulaire 23 et d'une zone 24 traversées par des micro-bulles provoquant une action de cavitation très importante sur le dépôt.

Le dispositif représenté à la figure 11 comprend une buse 13, dite à mémoire de forme, dont la particularité est de s'orienter dans des positions déterminées en fonction de sa température.

Cette buse 13 est constituée par plusieurs éléments : l'extrémité du conduit métallique 11 et un tube extérieur 33 réalisés en un alliage à mémoire de forme du type titane-aluminium, un tube 34 en matériau électriquement isolant intercalé entre l'extrémité du conduit 11 et le tube 33, et un tube périphérique 35 en matériau thermiquement isolant.

Le conduit 11 et le tube 33 sont reliés électriquement à leurs extrémités.

Un générateur électrique 36, à tension constante, et un rhéostat 37 permettent de faire circuler dans ces éléments un courant électrique d'intensité variable et de modifier ainsi leur température.

En faisant varier cette température de façon appropriée, on obtient des changements d'orientationprédéterminés de la buse 13 et, par voie de conséquence, des modifications de **directions du jet** supersonique.

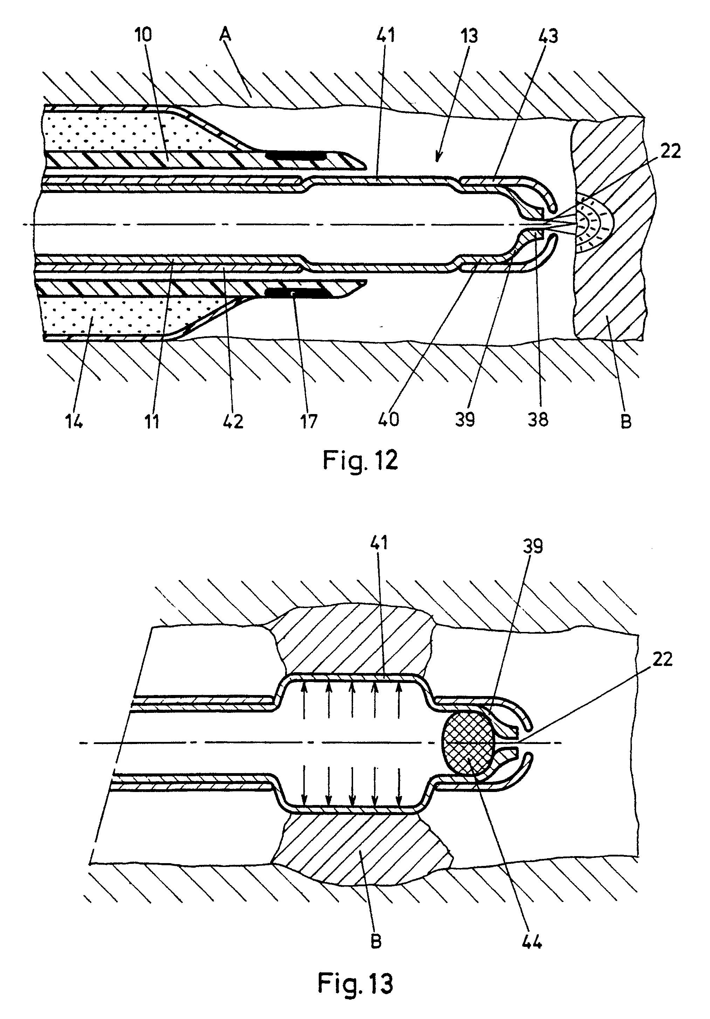

Le dispositif représenté à la figure 12 est constitué par un cathéter comprenant, comme celui de la figure 3 un tuyau souple 10, en matière plastique, un ballonnet intégré 14 e un collier radio-opaque 17, par un conduit métallique souple 11 et par une buse 13.

Le conduit 11 est réalisé en un alliage dont la limite de déformation permanente est très élévee ce qui lui permet de subir des allongements de l'ordre de 500 à 700% de la valeur initiale.

L'extrémité du conduit 11,qui constitue une partie de la buse 13, est formé d'un tronçon cylindrique 38, dont l'ouverture constitue l'orifice 22 de la buse 13, d'un tronçon tronconique 39, d'un tronçon cylindrique 40 et d'un tronçon cylindrique 41 de diamétres respectivement égal et légèrement plus grand que celui du conduit 11.

Le conduit 11 est contenu jusqu'au tronçon 41 dans une gaine de force en acier 42 et le tronçon 40 dans un embout de force 43, en acier, arrondi à son extrémité. Le tronçon cylindrique 41 est par conséquent libre de contrainte.

Ce dispositif s'utilise comme suit :

Après avoir amené l'extrémité du tuyau 10 dans le vaisseau A obstrué à proximité du dépôt B à éliminer, on gonfle le ballonnet 14, puis on met la buse en place en déplaçant le conduit 11 dans le tuyau 10.

On attaque ensuite le dépôt au moyen du jet supersonique engendré par la buse 13 en avançant progressivement dans le dépôt jusqu'à ce qu'il soit percé de part en part.

Ce résultat obtenu, on introduit dans le conduit 11 une bille 44, en matière plastique, de diamètre égal au diamètre intérieur du tronçon 40 de manière que cette bille 44, véhiculée par le liquide dont la pression a été réduite, vienne s'appliquer contre l'épaulement formé formé par le tronçon 39 et obturer l'orifice 22 (figure 13). Puis, on fait croître la pression du liquide ce qui provoque la **dilatation** du tronçon 41 qui vient en contact avec la paroi du trou. Le diamètre dilaté de ce tronçon peut atteindre une valeur de l'ordre de 0,6 à 1 mm soit trois fois son diamètre initial. Si nécessaire on peut encore agrandir ce trou en retirant la buse 13 et en terminant la dilatation au moyen du ballonet 14.

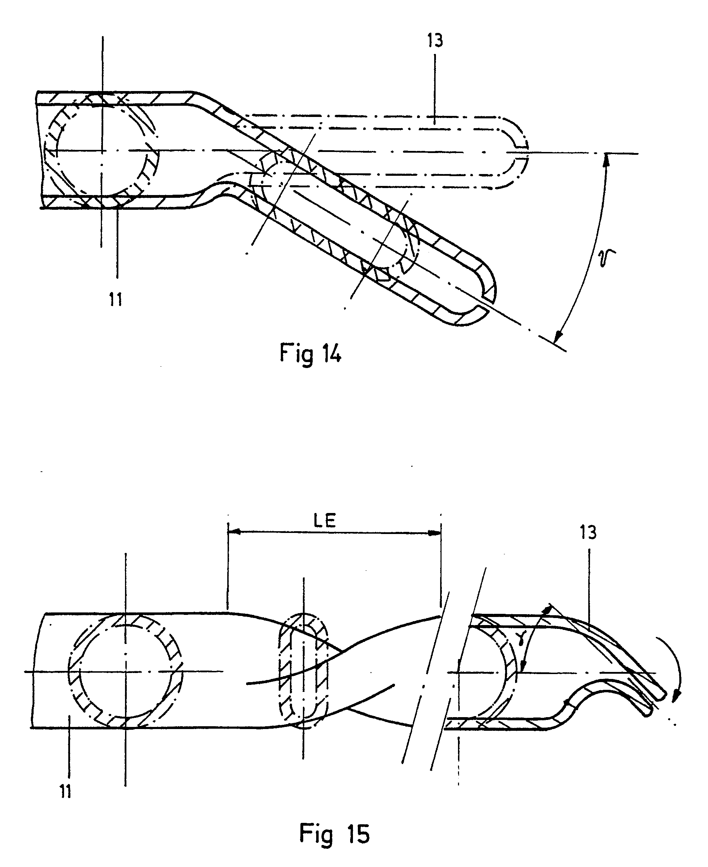

La buse représentée à la figure 14, dite buse à effet Bourdon, se distingue des précédentes par le fait que le tronçon terminal du conduit 11 a été aplati et légèrement courbé de manière d'une part à présenter une section elliptique et d'autre part à former un angle $\vartheta$ avec l'axe du conduit.

Lorsque la pression du liquide alimentant la buse augmente, cet angle $\vartheta$ diminue, car le tronçon en question a tendance à se redresser et, inversément, ce qui permet, en faisant varier la pression entre deux limites d'obtenir un balayage du jet et un accroissement de la surface de l'impa ct sur le dépôt.

Cette surface est encore augmentée en combinant l'effet Bourdon et une rotation du conduit autour de son axe.

La buse représentée à la figure 15, dite à effet spiral, se distingue des précédentes par le fait que le conduit 11 a été aplati et tordu le long d'un tronçon de longueur $L_E$ de telle sorte que ce tronçon présente d'une part une section elliptique et forme d'autre part une spirale.

De plus son extrémité circulaire forme un certain angle avec l'axe du conduit 11.

Lorsque la buse est alimentée en liquide sous pression, le tronçon spiral se redresse ce qui a pour conséquence de faire tourner cette extrémité autour de l'axe du conduit 11.

En faisant varier cette pression entre deux limites, on obtient un balayage circulaire et alternatif du jet ce qui accroit la surface de l'impact sur le dépôt.

Les avantages des dispositifs décrits sont les suivants :

1) Action stérilisante du liquide formant le jet dû à une très grande variation de pression dans un laps de temps très court
$\frac{\Delta p}{\Delta t} = 10^{13}$ à $10^{14}$ Pa / s.

2) Réglage très sensible et très précis de la profondeur d'action du jet en fonction de la pression et du temps d'usinage.

3) Force de recul de la buse de l'ordre de 0,4 N correspondant à une pression moyenne au niveau de l'impact du jet sur le dépôt de l'ordre de 125 N/mm$^2$.

4) Force d'impact du jet proportionnelle au sinus de l'angle d'impact sur le dépôt, donc maximum au niveau de ce dernier et minimum au niveau de la paroi du vaisseau.

5) Travail en temps réel par l'utilisation dz radio-contraste qui permet de visualiser l'action directe coupante ou érosive du jet pendant l'intervention.

6) Création d'un trou assez gros pour permettre au ballonet de rentrer dans celui-ci.

7) Pas de lésion thermique puisque la chaleur est éliminée par le liquide lui-même.

8) Pas de douleur ressentie par le patient consécutive à une action thermique.

9) Aucun risque de perforation des tissus lorsque l'on débouche à travers le dépôt (effet " dechanneling ").

10) Possibilité d'utiliser des sondes jusqu'à un diamétre de 0,15 mm ce qui permet d'aller dans des vaisseaux de très petit calibre.

L'invention n'est évidemment pas limitée aux dispositifs représentés et décrits.

En particulier la buse13 pourrait être constituée, dans une variante, par l'extrémité, fermée par un bouchon, du conduit 11, ce bouchon présentant un trou formant l'orifice 22 de la buse 13.

Des buses de ce dernier type pourraient être substituées aux buses représentées au dessin sans sortir du cadre de l'invention.

## Revendications

1. Dispositif chirurgical constitué par un cathéter comprenant un tuyau souple (10) et un conduit métallique souple (11) s'étendant dans ce tuyau (10), par une source de liquide sous pression (12) reliée à une extrémité de ce conduit (11), et par une buse (13) formant la seconde extrémité de ce conduit (11) et dont le diamètre est compris entre 20 et 70 microns, le tout de manière que la buse (13) projette un jet de liquide animé d'une vitesse relativement élevée, caractérisé par le fait que ladite source de liquide (12) alimente ladite buse (13) à une pression égale ou supérieure à $6.10^7$ Pa et par le fait que cette buse (13) forme un jet dont la vitesse initiale est égale ou supérieure au nombre de Mach.

2. Dispositif selon la revendication 1, caractérisé par le fait que ladite buse (13) est constituée au moins en partie par l'extrémité dudit conduit (11).

3. Dispositif selon la revendication 2, caractérisé par le fait que ladite buse (13) présente un tronçon de longueur $L_A$, dont la section décroit de façon continue, et un tronçon de longueur $L_B$ dont la section intérieure est constante et égale à celle de l'orifice (22) de la buse (13).

4. Dispositif selon la revendication 3, caractérisé par le fait que la longueur $L_A$ est comprise entre une et cinq fois le diamètre intérieur dudit conduit (11) et la longueur $L_B$ entre 0,2 et 5 fois le diamètre de l'orifice (22) de la buse (13).

5. Dispositif selon la revendication 2, caractérisé par le fait que ladite buse (13) est constituée entièrement par l'extrémité dudit conduit (11).

6. Dispositif selon la revendication 2, caractérisé par le fait que ladite buse (13) comprend au moins un tronçon d'un tube de force (25) engagé sur au moins une partie de la longueur dudit conduit (11).

7. Dispositif selon la revendication 2, caractérisé par le fait que ladite buse (13) comprend au moins un tronçon d'une gaine de protection (27) engagée sur au moins une partie de la longueur dudit conduit (11).

8. Dispositif selon la revendication 2, caractérisé par le fait que ladite buse (13) comprend une chambre de résonance (28).

9. Dispositif selon la revendication 8, caractérisé par le fait que ladite chambre (28) est formée par un rétrécissement de l'extrémité dudit conduit (11).

10. Dispositif selon la revendication 8, caractérisé par le fait que ladite chambre (28) est limitée d'une part par ledit conduit (11) et d'autre part par un tube métallique (29) engagé sur ledit conduit (11).

11. Dispositif selon la revendication 2, caractérisé par le fait que ladite buse (13) est une buse à mémoire de forme.

12. Dispositif selon la revendication 2, caractérisé par le fait que ladite buse (13) présente un tronçon (41) susceptible de se dilater sous l'action de la pression du liquide, et des moyens (44) pour obturer l'orifice (22) de la buse (13).

13. Dispositif selon la revendication 2, caractérisé par le fait que l'axe de l'orifice (22) de la buse (13) et l'axe dudit conduit (11) forment un angle $\beta$ compris entre 0 et 120 °.

14. Dispositif selon la revendication 2, caractérisé par le fait que ladite buse (13) comprend une pièce (31) s'étendant jusqu'à son orifice (22).

15. Dispositif selon la revendication 2, caractérisé par le fait que ladite buse (13) est constituée au moins en partie par l'extrémité, fermée par un bouchon, dudit conduit (11), ce bouchon présentant un trouconstituant l'orifice (22) de la buse (13).

16. Dispositif selon la revendication 2, caractérisé par le fait que l'extrémité du conduit (11) est aplatie et pliée de manière à former un certain angle avec l'axe du conduit (11).

17. Dispositif selon la revendication 2, caractérisé par le fait qu'au moins un tronçon dudit conduit (11) est aplati et torsadé et que la buse (13) forme un angle $\gamma$ avec l'axe du conduit (11).

## Claims

1. Chirurgical device constituted by a catheter comprising a flexible hose (10) and a flexible metallic conduit (11) extending within said hose (10), a source (12) of high pressure liquid connected to one end of said conduit (11), and a nozzle (13) forming a second end of said conduit (11) and having a diameter in the range of 20 to 70 micrometers, such that the nozzle (13) projects a liquid jet having a relatively high speed,
characterized in that said liquid source (12) feeds said nozzle (13) with a pressure equal to or higher than $6*10^7$ Pa, and in that said nozzle (13) forns a jet having a initial speed which is equal to or greater than the Mach number.

2. Device according to claim 1,
**characterized** in that said nozzle (13) is constituted, at least partly, by the end of said conduit (11).

3. Device according Co claim 2,
**characterized** in that said nozzle (13) has a section of a length $L_A$ having a continuously decreasing cross sectional area, and a section of length $L_B$ having an interior cross section which is constant and equal to that of the outlet (22) of said nozzle (13).

4. Device according to claim 3,
**characterized** in that said length $L_A$ is in the range of one and five times the internal diameter of said conduit (11), and in that said length $L_B$ is in the range of 0,2 to 5 times the diameter of the outlet (22) of said nozzle (13).

5. Device according to claim 2,
**characterized** in that said nozzle (13) is formed in its entirety by the end of said conduit (11).

6. Device according to claim 2,
**characterized** in that said nozzle (13) comprises at least a section of a reinforcement tube (25) in engagement over at least a portion of the length of said conduit (11).

7. Device according to claim 2,
**characterized** in that said nozzle (13) comprises at least a section of a shielding sheath (27) in engagement over at least a portion of the length of said conduit (11).

8. Device according to claim 2,
**characterized** in that said nozzle (13) comprises a resonance chamber (28).

9. Device according to claim 8,
**characterized** in that said chamber (28) is formed by a narrowing of the end of said conduit (11).

10. Device according to claim 8,
**characterized** in that said chamber (289 is defined on one hand by said conduit (11) and on the other hand by a metallic tube (29) in engagement on said conduit (11).

11. Device according to claim 2,
**characterized** in that said nozzle (13) is a nozzle with memory-shape.

12. Device according to claim 2,
**characterized** in that said nozzle (13) has a section (41) susceptible to expand under the action of the pressure of said liquid, and means (44) for obstructing the outlet (22) of said nozzle (13).

13. Device according to claim 2,
**characterized** in that the axis of the outlet (22) of the nozzle (13) and the axis of said conduit (11) form an angle in the range between 0 and 120°.

14. Device according to claim 2,
**characterized** in that said nozzle (13) comprises a piece (31) extending up to its outlet (22).

15. Device according to claim 2,
**characterized** in that said nozzle (13) is constituted at least partly by the end of said conduit (11) closed by a plug, said plug having a bore defining the outlet (22) if said nozzle (13).

16. Device according to claim 2,
**characterized** by the fact that the end of the conduit (11) is flattend and bent in a manner such as to form a certain angle with the axis of said conduit (11).

17. Device according to claim 2,
**characterized** in that at least a portion of said conduit (11) is flattend and twisted, and in that the

EP 0 232 678 B1

nozzle (13) forms and angle $\gamma$ with the axis of said conduit (11).

**Ansprüche**

1.  Chirurgisches Gerät mit einem Katheder, der einen biegsamen Schlauch (10) und eine sich in diesen Schlauch (10) erstreckende biegsame metallische Leitung (11) aufweist, mit einer Druckflüssigkeitsquelle (12), die mit einem Ende der Leitung (11) verbunden ist, und mit einer Düse (13), die das zweite Ende der Leitung (11) bildet und deren Durchmesser zwischen 20 und 70 Mikrometern liegt, derart, daß die Düse (13) einen Flüssigkeitsstrahl abgibt, dar mit einer relativ hohen Geschwindigkeit angetrieben ist,
dadurch **gekennzeichnet**, daß die Druckflüssigkeitsquelle (12) die Düse (13) mit einem Druck speist, der gleich oder größer als $6 * 10^7$ Pa ist, und daß die Düse (13) einen Strahl bildet, dessen Anfangsgeschwindigkeit gleich oder größer als Mach-Zahl ist.

2.  Gerät nach Anspruch 1,
dadurch **gekennzeichnet**, daß die Düse (13) zumindestens teilweise durch das Ende der Leitung (11) gebildet ist.

3.  Gerät nach Anspruch 2,
dadurch **gekennzeichnet**, daß die Düse (13) einen Abschnitt mit der Länge $L_A$, dessen Querschnitt in kontinuierlicher Weise abnimmt, und einen Abschnitt mit der Länge $L_B$ aufweist, dessen Innenquerschnitt konstant und gleich der der Öffnung (22) der Düse (13) ist.

4.  Gerät nach Anspruch 3,
dadurch **gekennzeichnet**, daß die Länge $L_A$ zwischen dem 1-fachen und dem 5-fachen des Innendurchmessers der Leitung (11) liegt und daß die Länge $L_B$ zwischen dem 0,2-fachen und dem 5-fachen des Durchmessers der Öffnung (22) der Düse (13) liegt.

5.  Gerät nach Anspruch 2,
dadurch **gekennzeichnet**, daß die Düse (13) vollständig durch das Ende der Leitung (11) gebildet ist.

6.  Gerät nach Anspruch 2,
dadurch **gekennzeichnet**, daß die Düse (13) zumindestens einen Abschnitt eines Verstärkungsrohres (25) umfaßt, das über zumindestens einen Teil der Länge der Leitung (11) mit dieser in Eingriff steht.

7.  Gerät nach Anspruch 2,
dadurch **gekennzeichnet**, daß die Düse (13) zumindestens einen Abschnitt einer Schutzhülle (27) aufweist, die über zumindestens einen Teil der Länge der Leitung (11) mit dieser in Eingriff steht.

8.  Gerät nach Anspruch 2,
dadurch **gekennzeichnet**, daß die Düse (13) eine Resonanzkammer (28) aufweist.

9.  Gerät nach Anspruch 8,
dadurch **gekennzeichnet**, daß die Kammer (28) durch eine Einschnürung des Endes der Leitung (11) gebildet ist.

10. Gerät nach Anspruch 8,
dadurch **gekennzeichnet**, daß die Kammer (28) einerseits durch die Leitung (11) und andererseits durch ein Metallrohr (29) umgrenzt ist, das auf der Leitung (11) in Eingriff steht.

11. Gerät nach Anspruch 2,
dadurch **gekennzeichnet**, daß die Düse (13) eine Düse mit Formgedächtnis ist.

12. Gerät nach Anspruch 2,
dadurch **gekennzeichnet**, daß die Düse (13) einen Abschnitt (41) aufweist, der sich unter der Wirkung des Druckes der Flüssigkeit dehnen kann, und daß Einrichtungen (44) zum Verschließen der Öffnung (22) der Düse (13) vorgesehen sind.

9

13. Gerät nach Anspruch 2,
dadurch **gekennzeichnet**, daß die Achse der Öffnung (22) der Düse (13) und die Achse der Leitung (11) einen Winkel $\beta$ bilden, der zwischen 0 und 120° liegt.

14. Gerät nach Anspruch 2,
dadurch **gekennzeichnet**, daß die Düse (13) ein Teil (31) aufweist, das sich bis zur Öffnung (22) der Düse erstreckt.

15. Gerät nach Anspruch 2,
dadurch **gekennzeichnet**, daß die Düse (13) zumindestens teilweise durch das durch einen Stopfen verschlossene Ende der Leitung (11) gebildet ist, wobei dieser Stopfen eine Bohrung aufweist, die die Öffnung (22) der Düse bildet.

16. Gerät nach Anspruch 2,
dadurch **gekennzeichnet**, daß das Ende der Leitung (11) abgeflacht und derart gebogen ist, daß es einen bestimmten Winkel mit der Achse der Leitung (11) bildet.

17. Gerät nach Anspruch 2,
dadurch **gekennzeichnet**, daß zumindestens ein Abschnitt der Leitung (11) abgeflacht und verdreht ist, und daß die Düse (13) einen Winkel $\gamma$ mit der Achse der Leitung (11) bildet.

Fig 1

Fig 2

Fig. 3

Fig. 4

Fig. 5

Fig. 6

Fig. 7

Fig. 8

Fig. 9

Fig. 10

Fig. 11

Fig. 12

Fig. 13

Fig 14

Fig 15